# EUROPEAN PATENT APPLICATION

(11) **EP 1 724 338 A1**
(43) Date of publication of application: **22.11.2006**
(21) Application number: 05104267.9
(22) Date of filing: 19.05.2005
(51) Int. Cl.: C12N 7/02, C12N 7/06, A61K 39/12

(54) **Methods for the production of a whole-inactivated West Nile Virus vaccine**

(71) Applicant: Crucell Holland B.V., 2333 CN Leiden (NL)
(72) Inventor: de Vocht, Marcel, Leo, 3445 TE Woerden (NL); Backus, Harold Hubertus Jacobus, 2324 LA Leiden (NL); van Corven, Emile Johannes Josephus Maria, 3583 VS Utrecht (NL); Lagerwerf, Fija, 2353 WZ Leiderdorp (NL); Heemskerk, Evert, 2332 TT Leiden (NL); Uytdehaag, Alphonsus Gerardus Cornelis Maria, 3452 EH Vleuten (NL)
(74) Representative: Klein, Bart

(57) **Abstract**

The invention relates to methods for the purification of flaviviruses, preferably West Nile Viruses. The invention also relates to methods of inactivating West Nile Viruses as well as to formulating and adjuvating West Nile Virus vaccines, wherein said formulations are suitable for use in humans.

## Description

### FIELD OF THE INVENTION

The invention relates to the field of vaccines. In particular, it relates to down-stream processes of producing a whole-inactivated West Nile Virus vaccine, including methods for inactivation of the virus and formulating the produced vaccine. The vaccines are particularly useful in the vaccination of mammals, especially humans.

### BACKGROUND OF THE INVENTION

West Nile Virus is a member of the family of flaviviridae, which contains three genera: the flaviviruses, the pestiviruses and the Hepatitis C viruses. The flavivirus genus comprises more than 60 highly related viruses including several human pathogens of global and local epidemiological importance, with most of them being transmitted by arthropod vectors. With a combined toll of hundreds of millions of infections around the world annually, yellow fever virus, Japanese encephalitis virus, St. Louis encephalitis virus, Murray Valley encephalitis virus, tick-borne encephalitis virus, dengue virus, and West Nile Virus continue to be in the focus of epidemiological surveillance worldwide. While the availability of an efficient vaccine and control of mosquito vectors have resulted in significant improvement of the epidemiological situation in yellow fever, other existing as well as emerging flavivirus-associated diseases, for which vaccines are not yet available, continue to challenge experimental virology (reviewed by Chang et al. 2004).

West Nile Virus was first identified in 1937 in the West Nile district in Uganda and has now been recognized as the most widespread of the flaviviruses, with a geographic distribution in Africa, Asia, Europe, Australia and North America. Outbreaks have been reported in Russia, Israel, Romania, and the United States. The virus was found to have caused infections in persons in over 40 different states in the US. Symptoms vary from fever and headache to coma, while in 15% of the cases the disease progresses to a more severe state (e.g., West Nile encephalitis), which can lead to death. Besides infecting humans, West Nile Viruses are also known to infect horses and several bird species and cause severe illness and death. The outbreak in New York in 1999 started with massive death among crows and several lethal cases in horses.

Several approaches were followed in the art to counteract the infection and resulting illnesses brought about by flaviviruses. One proposed approach was to treat individuals with chemical compounds, such as ribavirin and nucleoside analogues, and biologicals such as interferon alpha-2b and/or helioxanthin (WO 00/10991; WO 02/15664; US patent no. 6,306,899). Others have focused on the development of vaccines containing for instance chimeric flaviviruses, (manipulated) yellow fever viruses for cross-vaccination, sub-viral particles, replication defective flaviviruses, (naked) nucleic acid, recombinant sub-units (envelope proteins), or poxviruses containing flavivirus antigens (see Arroyo et al. 2001; Wang et al. 2001; Chang et al. 2001; WO00/12128; WO01/03729; WO02/72036; WO99/26653; WO99/63095; WO01/60315; WO02/68637; EP0869184; WO00/14245; WO02/74963; EP0691404; WO98/37911; WO01/39802; WO01/60847; WO03/063902; WO02/56824; WO02/81621; WO2004/045529; WO03/060088; EP0102228; EP0872553; US 6,184,024; US 5,514,375; US 5,744,140; US 5,744,141; US 6,416,763; US 6,432,411; US 5,494,671; US 6,258,788; and US 6,676,936). One veterinary vaccine containing inactivated West Nile Viruses was approved in 2001, solely for the use in horses (see e.g., WO 03/061555). In Israel, an approach was taken to produce a West Nile Virus strain isolated from geese (Goose Israel 1998) in mouse brains, to inactivate it by formaldehyde and to apply the vaccine in geese flocks (Malkinson et al. 2001). The Israelian authorities approved this veterinary vaccine in 2001 for use in geese flocks. A novel method for producing West Nile Viruses using an adenovirus E1-transformed human cell line was disclosed in WO 2004/042042.

Most of the vaccines produced thus far have been developed for animals such as geese, birds and horses. The requirements for such vaccines are not as strict as they are for vaccines that are to be used in humans, with respect to safety, efficacy and dosage. The whole-inactivated vaccine that was approved for geese in Israel was based on a formalin-inactivated virus, where the virus particles were produced on mouse brains. The vaccine that was approved for horses was also made through formalin-inactivation and formulated in mineral oil. Many of the processes and formulations that are used for vaccines that are used in animals cannot be applied for vaccines that are suitable for the use in humans, as more stringent safety issues are at stake. As there is no available vaccine against West Nile Virus in man, there is a need for a well-defined, safe, straightforward, fast and reliable process that yields high titers of viruses, for the production of whole-inactivated West Nile Virus vaccines that can be used in clinical trials and vaccination programs for humans.

### BRIEF DESCRIPTION OF THE DRAWINGS

Figure 1 shows the preferred purification scheme according to the present invention.
Figure 2 depicts the elution profile from the Cellufine sulfate chromatography column. The flow through peak is indicated on the left, whereas the virus peak is indicated on the right. The thin line indicates the conductivity, while the fat line is the elution profile at OD₂₈₀.
Figure 3 shows the elution profile from the group separation chromatography column. The thin line represents the conductivity, while the fat line represents the elution profile at OD₂₈₀, with the virus peak and the contaminating residues indicated.

### SUMMARY OF THE INVENTION

The present invention relates to novel processes for the production of a West Nile Virus vaccine, wherein the vaccine is in principle suitable for use in clinical trials that involve humans. The invention further relates to methods for inactivation of the produced West Nile Viruses as well as to formulations for the produced virus particles obtained according to the processes of the present invention.

The invention relates to a new method for the inactivation of a flavivirus, comprising subjecting said flavivirus to beta-propiolactone (BPL) for an appropriate period of time to yield a solution in which no live flavivirus can be detected. The preferred flavivirus that is being inactivated is West Nile Virus.

The invention further relates to a method of purifying a flavivirus, wherein the virus is being inactivated first according to the methods of the present invention and further subjected to affinity chromatography and/or group separation chromatography.

The invention also relates to a West Nile Virus vaccine comprising a whole-inactivated West Nile Virus, wherein said West Nile Virus is formulated in a solution that is pH-controlled and further comprises a salt, a detergent and a pharmaceutically acceptable carrier. Preferably, said West Nile Virus vaccine comprises an adjuvant, preferably in the form of an aluminum-based adjuvant.

### DETAILED DESCRIPTION

The present invention intends to solve at least some of the problems outlined above and relates to processes for the production of a safe, efficacious and reliable vaccine against flavivirus-, and especially West Nile Virus infections in humans. It discloses the entire process from growth in suitable cells until the filling of the final product in a suitable formulation. Although the examples disclosed herein are limited to the production, inactivation, purification and formulation of West Nile Virus, it is to be understood that the disclosed methods may also be applied to other similar flaviviruses. The methods of the present invention can therefore add to the production of vaccines based on other flaviviruses that may be useful in the therapeutic or prophylactic treatment of diseases resulting from non-West Nile Virus flavivirus infections.

For the initial production of West Nile Viruses, an adenovirus E1-transformed cell line may be used as disclosed in WO01/38362 and WO2004/042042. The cells that are being applied herein as an example for such production are being marketed by Crucell Holland B.V. (Leiden, the Netherlands) as PER.C6® cells, which cells were also deposited at the European Collection of Cell Cultures (ECACC, Porton Down, Wiltshire, SP4 0JG, United Kingdom) under deposit no. 96022940. The generation of the cells has been described in detail elsewhere (see a.o. US 6,033,908 and EP 0833934 B1). The invention is not limited to the use of the adenovirus E1-transformed cells and the PER.C6^{®} cells serve as an example of how such viruses may be produced upstream of the methods according to the invention. Clearly, any host cell that is suitable for the propagation of West Nile Viruses, or flaviviruses in general, can be used for the production of the virus particles that are subsequently ready to be purified according to the methods of the present invention. Examples of other cells that are or may be used in the field for the propagation of West Nile Viruses are other E1-transformed cells such as 293 and 911 cells, Vero cells, MDCK cells and mouse embryo fibroblasts. The advantages of using suspension-growing cells under serum-free conditions have been described in W02004/042042. PER.C6® cells are cells that can be cultured under serum-free conditions and in suspension, free from carriers.

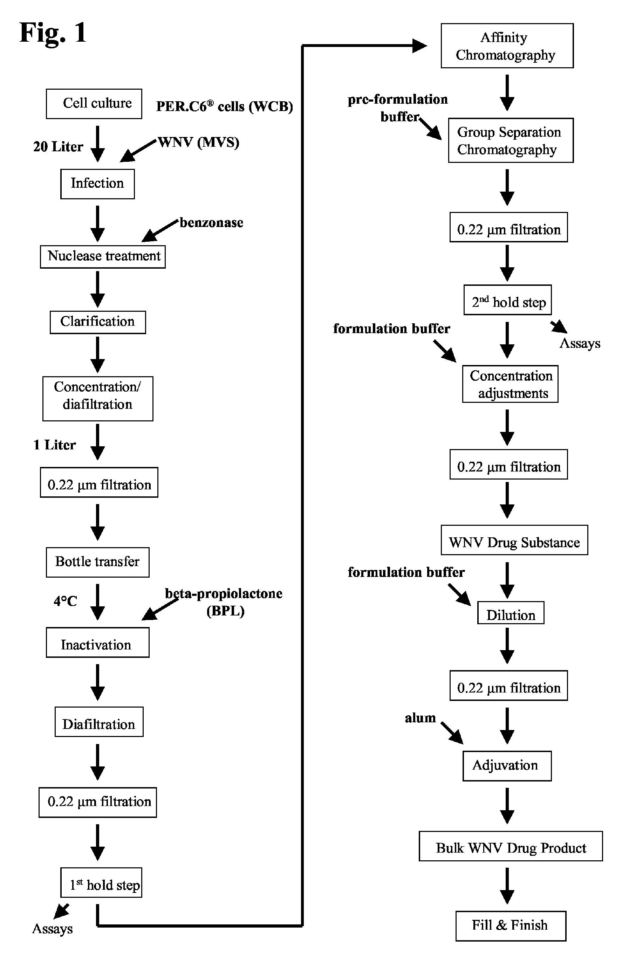

The system that is preferably used to infect cultured cells with WNV, to treat them with nuclease and subsequently clarify, filter and inactivate the produced particles is outlined schematically in figure 1 on the left. After verification that all particles were indeed inactivated during a first hold step, the system continues with chromatography, filtration and adjuvation as outlined in figure 1 on the right.

Inactivation of flaviviruses including West Nile Viruses using formaldehyde has been described in the art. This process is time-consuming as it lasts for several weeks at low temperatures to fully inactivate all viruses. The invention relates to a novel process in which WNV is whole-inactivated by the use of another known inactivator compound, beta-propiolactone. This enables a much faster method for inactivation. It is disclosed herein that by using BPL, a completely inactivated batch of WNV can be obtained. The different compounds have different modes of action. BPL is an alkylating agent while formaldehyde is a cross-linker. It would therefore be relatively easy for the skilled person to distinguish between WNV products inactivated by formaldehyde, and WNV products inactivated by BPL. Thus, the inventions also relates to the whole-inactivated viruses obtained by the methods of inactivation according to the invention.

The affinity chromatography step is preferably applied for the purpose of disrupting certain protein interactions that occur between contaminating proteins and the virus particles. This disruption may occur by the use of a high salt elution buffer. The high salt concentration of the solution ensures the removal of the contaminating (and previously bound) proteins in a subsequent purification step, such as the group separation step as disclosed herein. Therefore, the invention also relates to a method of disrupting protein interactions between a flavivirus, preferably a West Nile Virus, and host cell proteins by applying a chromatography step in which a high salt elution buffer is used. The person skilled in the art is able to understand the meaning of the term 'high salt' as normal salt concentrations are unsuitable for eluting proteins/particles from an affinity chromatography column. It can thus be determined with which concentration of salt the elution takes place. This concentration and higher is therefore referred to as 'high salt'. Clearly, it depends on the column material and the bound proteins and particles whether a concentration is high (elution occurs) or whether a concentration is not high (no elution occurs). Preferably, a concentration of about 1 to 2 M is used, whereas it is even more preferred to use a concentration of approximately 1.5 M NaCl to elute the particles from the column and thereby also disrupt the interaction between the contaminating (sticking) proteins and the viral particles.

The invention relates to a method for inactivating a flavivirus, comprising subjecting said flavivirus to beta-propiolactone (BPL) for an appropriate period of time to yield a solution in which no live flavivirus can be detected. In a preferred embodiment, said flavivirus is a West Nile Virus. Said virus may be produced on different systems, such as mouse brain; however, preferably, said flavivirus is grown on cultured cells in vitro as such a setting provides a safer and more controlled way of production. To remove unwanted cellulair nucleic acids that may be released by the host cells, it is preferred that the mixture of medium comprising the flaviviruses produced on said cells in tissue culture is treated with a nuclease, preferably benzonase. Although nuclease treatment may also be applied after clarifying the solution, in a preferred setting, the benzonase treatment takes place after infection and before any further processing of the solution. It is thus to be understood that preferably said benzonase treatment takes place before clarification, diafiltration and/or inactivation. In a preferred embodiment said benzonase treatment is being performed just after the viruses have been produced on said cells in said tissue culture, preferably in the same medium comprising the virus particles as well as all contaminating host cell products.

The invention also relates to methods of inactivating a flavivirus, further comprising one or more of the steps of clarification and/or concentration/diafiltration.

Preferably, in said methods for inactivating a flavivirus, said BPL is present in a range of 0.01% to about 5%, preferably in a range of about 0.025% to about 1%, more preferably in a concentration of about 0.025% to about 0.5%, and most preferably in a concentration of about 0.1%. The invention discloses an inactivation method wherein the BPL treatment is for a sufficient amount of time that no live virus can be detected afterwards. As disclosed herein, the person skilled in the art can perform different assays to determine whether any live virus is still present. If there is, the amount of time was not sufficiently long enough to yield a whole-inactivated flavivirus batch. Thus, in view of the description as provided herein, the skilled person is able to determine what 'an appropriate period of time' as claimed herein indicates and actually means, as any live virus particles may be determined by IVA or suckling mice assay as described intra. In a preferred embodiment, the invention relates to a method according to the invention, wherein said inactivation by BPL takes place during a period of about 5-72 hr, preferably during a period of about 10-48 hr, more preferably during a period of about 18-44 hr, and most preferably during a period of about 36-44 hr.

The invention also relates to further down-stream processes that are to be performed after the nuclease (exemplified by benzonase) treatment and the inactivation procedure. The invention discloses novel ways of purifying flaviviruses, and preferably West Nile Viruses. In another preferred embodiment, the method of the invention further comprises a step of binding said inactivated flavivirus to an affinity chromatography resin.

The invention also relates to a method of purifying a West Nile Virus comprising the step of binding an inactivated West Nile Virus to an affinity chromatography resin. For this particular aspect, the West Nile Virus may also have been inactivated through other means, such as formalin-inactivation. In a preferred embodiment, said affinity chromatography is either followed or preceeded by a group separation chromatography step. It was found by the inventors of the present invention that elution from the affinity resin (preferably Cellufine sulfate) using high salt buffer results in virus particles that are substantially free from (sticking-, or in other ways interacting and normally co-purified) contaminating proteins. It is to be understood that said high salt step may also be performed in another phase of the process, thereby resulting in virus particles that do not interact with contaminating proteins. Thus, the invention also relates to a purification method for West Nile Viruses wherein chromatography steps are omitted and a high salt step ensures a proper separation of virus particles from contaminating material, which can be separated for instance in a concentration/diafiltration step using an appropriate cut-off.

When a chromatography resin is used to bind the (inactivated) flaviviruses, most preferably Cellufine sulfate is used. Other preferred resins that are useful are the anion exchange resins Q-sepharose-FF, Q-sepharose-HP, Q-sepharose-XL, Q-ceramic hyper DF and ANX-sepharose-FF. In a preferred aspect of the chromatography purification method, the methods of the present invention comprise the step of eluting the bound flavivirus by a high salt concentration in the elution buffer having a preferred pH of 7.5. Other pH values may also be used (preferably between 7.0 and 8.0), depending on the remaining components of the elution buffer as well as on the amount of contaminants co-eluted. Preferably, the high salt concentration is about 1 to about 2 M NaCl, preferably about 1.5 M NaCl. The skilled person can easily determine the useful concentration for elution of the particles from the resin, and thereby determine what should be understood by a 'high' salt concentration. Concentrations wherein said particles are not eluted are considered too low. Concentrations wherein certain proteins that interact with the virus particles (presumably certain heat shock proteins) do not release from the particles are also considered too low. Clearly, different salts may be used to reach a 'high' salt concentration. The person skilled in the art understands that a 'high' salt concentration is that concentration with a certain salt having sufficient ionic strength to disrupt the unwanted interactions. The preferred salt is NaCl.

In yet another preferred embodiment, the methods of the present invention comprise a step of subjecting said (inactivated) flavivirus to a group separation chromatography step. More preferably, the affinity chromatography step preceeds said group separation chromatography step. Also preferred are methods, further comprising one or more diafiltration steps.

The invention also relates to a flavivirus obtained according to a method of the present invention. Preferably, said obtained flavivirus is a West Nile Virus. It is to be understood that the flaviviruses obtained according to the methods of the present invention are different from flaviviruses obtained according to methods known in the art as the BPL-inactivation is a different process from the generally used formalin-inactivation. The skilled person is able to distinguish between the two inactivated versions by several analytical methods known in the art.

The invention further relates to a West Nile Virus vaccine comprising a whole-inactivated West Nile Virus, wherein said West Nile Virus is formulated in a solution that is pH-controlled and further comprises a salt, a detergent and a pharmaceutically acceptable carrier. Possible pharmaceutically acceptable carriers are well-known in the art and not further elaborated upon. The meaning of the terms detergents and salts are also generally known. In a preferred embodiment, the invention relates to a West Nile Virus vaccine, further comprising an adjuvant. More preferably, said adjuvant is an aluminum-based adjuvant. And even more preferably, said adjuvant is aluminum hydroxide. In yet another preferred aspect, the salt in the vaccine formulation according to the invention is NaCl, whereas the preferred detergent is Tween-80.

### EXAMPLES

The entire inactivation/purification/formulation process as outlined in the following examples is represented schematically in figure 1.

### Example 1. Production of West Nile Virus on PER.C6® cells and subsequent nuclease treatment using benzonase

PER.C6® cells were first thawed from a working cell bank (WCB) kept at -65° and seeded at a concentration of 0.33x10⁶ viable cells/ml in 15 ml serum-free medium (Biochrome) and subsequently cultured in suspension shaker flasks and wave bags up to a volume of 2x10 liter and a cell density of 1.0x10⁶ cells/ml (T = 35°C and pH 6.5-7.5). Then, wild type West Nile Viruses (strain NY99: New York 99) were taken from a Master Virus Seed stock (MVS) kept at -65°C and infection was performed with a multiplicity of infection (moi) of 5x10⁻³ vp/cell. Infection was continued for 6x24 h. To remove unwanted nucleic acids (mainly in the form of host cell DNA and RNA), a nuclease was subsequently added in the form of benzonase (Merck), which step was performed with 100,000-500,000 Units/10L concentration for 0.5-1 hr at T = 37°C. The concentration of WNV after infection and during the subsequent down-stream process was determined by an WNV E protein specific ELISA assay discussed below.

### Example 2. Inactivation of the produced West Nile Viruses

The infected cell culture of the previous example was treated further as follows to ensure fully inactivated virus. The culture supernatant was first clarified by removal of the cells by filtration over a 3 µm depth filter (Millipore) followed by a filtration over a 0.80/0.45 µm membrane filter (Sartorius) into a clean bottle, thereby obtaining 20 liter of clarified culture supernatant.

Then, the harvest was concentrated 20x in a tangential flow filtration (TFF) system with a hollow fiber filter (300 kDa MWCO, GE Healthcare). Through this, the volume for the subsequent diafiltration and inactivation step results in approximately 1 liter. This concentrate was then diafiltered 10x with the same TFF-unit with inactivation buffer (50 mM Hepes, 150 mM NaCl, pH 7.9). During this step, low molecular weight impurities are removed and the medium is replaced with buffer that is suitable for inactivation.

To remove potential aggregates of the virus and to reduce the bioburden level, the concentrate was filtered over a 0.22 µm filter, by connecting the retentate bottle to a filtration setup with a 0.80/0.45 µm membrane filter (Sartorius) and a 0.22 µm stacked disc filter (Millipore). The diafiltered virus solution was pumped through the filters and the volume of the filtered product was determined accurately by weighing.

Then, the filtered material was transferred into a precooled water-jacketed spinner flask and the material was cooled to 4°C. Separately, a 10% beta-propiolactone (BPL, Ferak, Berlin, Germany) was prepared freshly by dilution in cold water. The 10% BPL solution was then added to the material in the spinner flask to result in a BPL concentration of about 0.1%. Inactivation by BPL was continued for 36-44 hr with gentle stirring at 4°C, after which a sample was taken. A sample was also taken at 18 hr in which the activity of the BPL was quenched by addition of excess thiosulfate. The absence of live virus of this sample was determined by an *in vitro* amplification (IVA) assay and an *in vivo* suckling mouse assay (see below).

The solution comprising the supposedly inactivated West Nile Virus particles was diafiltered 10x with a hollow-fiber unit (400 kDa membrane, Spectrum) to remove residual BPL and to exchange the buffer for a chromatography-binding buffer (50 mM Hepes, 150 mM NaCl, pH 7.5). To reduce the bioburden level, the material was again 0.22 µm filtered: The diafiltration bottle was connected to a filtration setup with a 0.22 µm stacked disc filter (Millipore), and the virus solution was pumped through the filters. The supposedly inactivated bulk was stored in a first hold step for about 5 weeks at 4°C to allow the inactivation testing assays to be performed. Before storage, a sample was taken for the assays.

The IVA assay was designed to amplify live virus potentially present in samples after BPL inactivation to a level where subsequent testing of culture medium in suckling mice will result in a positive outcome. Samples that do show a cytopathological effect (CPE positive) are to be tested in an immonufluorescence (IF) assay. If the IF is positive for WNV, the sample is regarded as not inactivated, whereas when the IF assay result is negative, although CPE occurred, the IVA is regarded as invalid. In both cases the sample is not tested in suckling mice, preventing unnecessary animal experiments.

The IVA assay for the detection of infectious WNV in BPL inactivated vaccine material has been qualified as a limit test. The assay is able to detect one TCID₅₀ per inoculated volume with a confidence of 99%. Furthermore, the assay is specific for WNV when combined with a confirmatory IF procedure when sample flasks show up CPE positive. The IVA assay is performed by culturing 6-7x10⁶ attached cells/ml in a T175 flask. The following day, 2.2 ml inactivated virus sample is added to a total volume of 53 ml medium. CPE is scored visually after 10-14 days of culturing at 37°C.

The *in vivo* suckling mouse inactivation test was developed for the detection of WNV infectious particles in whole inactivated WNV vaccine material. The assay comprises an IVA step (see above), followed by injection of 30 µl supposedly inactivated virus sample into suckling mice brains (30 µl/mouse). If the IVA did not show any CPE, the supernatant was tested in suckling mice. Also this *in vivo* assay is being qualified as a limit test, including a limit of detection (LOD) and intermediate precision. For all batches at least 9 mice were injected. A batch was regarded as inactivated when all mice survived for 14 days. The preliminary LOD is estimated to be 1.5 TCID₅₀/30 µl per mouse. The intermediate precision is defined as the standard deviation from ~25 experiments around the average LOD with varying operators and challenge virus batches. This standard deviation is estimated at 0.9 TCID₅₀, and the range in which 95% of the experiments are expected to fall within is 0.3 to 0.5 TCID₅₀.

In all six runs that were performed in full, no CPE was determined in the IVA assay, whereas in the subsequent suckling mouse assays none of the mice died in the period given after injection. Thus, both assays revealed that all viruses (also in the intermediate steps) were indeed inactivated by the incubation with BPL in the period used. It was concluded that BPL inactivation is a proper method to fully inactivate WNV viruses to ultimately produce whole-inactivated WNV vaccines.

### Example 3. Purification of whole-inactivated West Nile Viruses

After the first hold step, when it appeared that inactivation was indeed complete, the virus solution was subjected to chromatography. Several resins can be tested to have no E protein of WNV in the flow through, wherein the E protein could be eluted with at least 1 M salt and no E protein would be present in the wash step(s): Sephacryl S-300, Sepharose 6-FF, SP Sepharaose, S-ceramic hyper D F, Fractoprep SO3-, Hydroxy apatite, Fractogel EMD DEAE, Fractoprep TMAE, Source 30-Q, DEAE sepharose, DEAE 650M, Super Q 650M, QAE resin, and DEAE-ceramic hyper D F. The preferred resins that were tested were the anion exchange resins Q-sepharose-FF, Q-sepharose-HP, Q-sepharose-XL, Q-ceramic hyper DF and ANX-sepharose-FF. The most preferred resin is Celluline sulfate, which is a rigid cellulose matrix with a low concentration of sulfate ester. It comprises low-density cation exchange groups and it provides affinity (heparin) binding properties. Importantly, endotoxins and DNA do preferably not bind to the selected column. The use of Cellufine sulfate chromatography for the purification of viruses has been described in the art: US 4,724,210 (Influenza), US 4,515,714 (Hepatitis B surface antigen), US 4,725,547 (Rabic virus), US 4,725,546 (Japanese Encephalitis virus, a flavivirus) and US 4,724,146 (Herpes simplex virus subunit).

Several conditions were tested with respect to pH, salt concentrations and phosphate concentrations. When an increasing concentration of salt was used to elute the proteins and viruses, the virus particles either eluted together with the initial contaminating proteins or in a separate peak at higher salt concentrations. It was found that when a concentration of 1.5 M NaCl was used in a block gradient (increasing the salt concentration suddenly to 1.5 M), the virus eluted in a single peak. It is known in the art that certain protein have a strong affinity to flaviviruses. Recently, Reyes-Del Valle et al. (2005) have shown that heat shock protein 70 and -90 bind strongly to Dengue virus (a flavivirus) and that this interaction can be disrupted by using a high salt buffer (1.5 M). The inventors of the present invention have now found that these heat shock proteins (produced by the host cell) do also interact with WNV and that these strong interactions can be disrupted by using the same concentration of salt. This feature is being applied by eluting the bound proteins and virus particles from the Cellufine sulfate (affinity) column using the high salt buffer. Although the heat shock proteins (and other contaminants) elute in the same fractions as the virus particles, it is now possible to separate these proteins and virus particles in a subsequent group separation chromatography step. Although the best mode for purification (as contemplated thus far) is described below, it may be that the use of the high salt step would enable to disrupt the interactions at a much earlier stage in the entire process, for instance before the diafiltration step (see above) that is being used before inactivating the viruses. If this indeed turns out to be a feasible procedure, one may even omit the chromatography steps as the viruses and contaminating proteins are already separated in the diafiltration step by using a cut off filter of 300 kD. Although this requires further optimisation of the conditions, the invention also relates to a process for purifying flaviviruses, preferably West Nile Viruses, in which no chromatography step are being used and wherein the viruses are separated from contaminating proteins in a diafiltration step using high salt, preferably a salt concentration between 1 and 2 M, more preferably of about 1.5 M. The invention thus also relates to vaccines comprising a whole-inactivated WNV that is being purified without chromatography and wherein the contaminating proteins have been removed from the solution comprising the virus particles before inactivation. Inactivation is also preferably performed using beta-propiolactone.

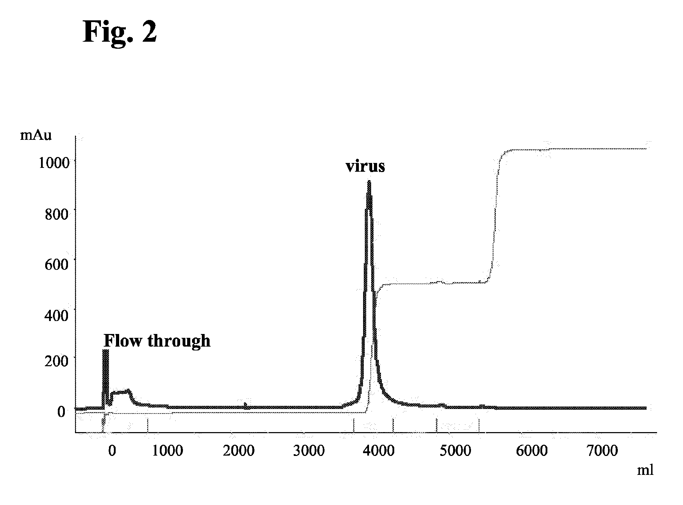

Here, it is described how the WNV particles were purified to an extent that the product can be used in clinical settings as it provides a sufficiently clean and safe final product. The inactivated WNV product was first bound in an affinity chromatography step to the Cellufine Sulfate column (Chisso, Japan) to which some contaminating host-cell proteins and remaining DNA impurities do not bind. After the binding of the product, the column was washed with binding buffer (50 mM Hepes, 150 mM NaCl, pH 7.5), followed by an elution with (high salt) elution buffer (50 mM Hepes, 1.5 M NaCl, pH 7.5). The elution peak (see figure 2 for the elution profile) was collected in a separate bottle. The elution buffer contains a high concentration of salt (1.5 M NaCl). This concentration may be sufficient for the separation of certain contaminating proteins from the virus particle, such as removal of heat shock protein and the like. The high salt concentration, resulting from the cellufine sulfate chromatography step is therefore in itself an important contributor to the purification state of the final product.

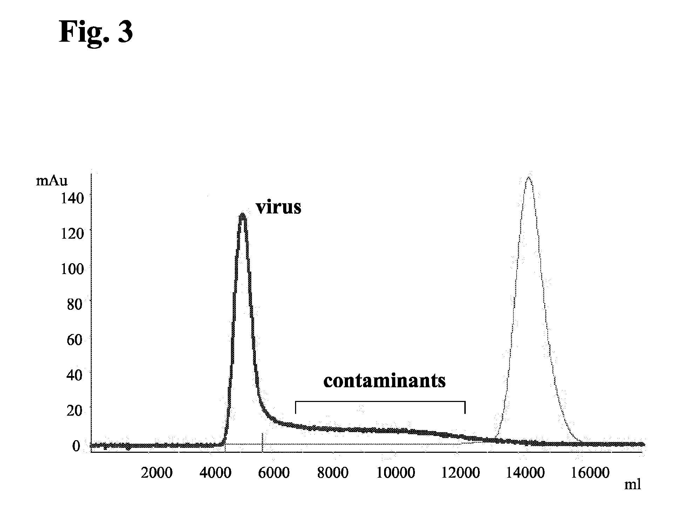

Remaining impurities were then preferably removed by a subsequent group separation chromatography step based on differences in size: large particles (such as the inactivated virus) elute first, whereas the relatively small particles (such as proteins) are retarded. Furthermore, during this step, the buffer is changed to 20 mM Tris, 110 mM NaCl, pH 7.4, which is the formulation buffer without Tween-80 (see below). The group separation chromatography column was pre-equilibrated with the formulation buffer without Tween-80 before loading of the high-salt eluted product from the affinity column. The product was eluted from the group separation column before the impurities elute (see figure 3 for the elution profile). It should be noted that, due to the high salt concentration resulting from the cellufine sulfate step many contaminants are removed by this further group separation step that would have remained attached to the virus particles if no high salt was applied. It is therefore highly preferred to use high salt in the preferred earlier step of using cellufine sulfate.

To reduce the bioburden level, the material was again filtered with a 0.22 µm stacked disc filter (Millipore), after which the filtered material was stored for about a week at 4°C in a second hold step during which the concentration of the product was accurately determined by using an ELISA wherein an antibody was used directed against the E protein of WNV. This ELISA is preferably used throughout the purification scheme, as with every step the loss of virus particles can be determined accurately.

For this, Greiner 96-wells plates were coated overnight at 4°C with 0.4 pg/ml mouse anti-E protein monoclonal antibody 7H2 (Bioreliance). The next day, wells were washed 3 times with 300 µl of 0.0005% Tween-20 (v/v) in PBS (wash buffer). This washing procedure was performed after each incubation period unless described otherwise. Plates were blocked with 2% gelatine blocking buffer (Sigma) for 2 h at room temperature (RT). Subsequently, WNV samples were diluted in PBS supplemented with 0.0005% Tween-20 (v/v) and 1% non-fat dry milk (w/v; Biorad) to concentrations of 5 to 345 EU/ml. 100 µl of each sample was added per well and incubated for 1 h at RT. Semi-purified inactivated WNV served as reference for preparation of the standard curve and 2 other inactivate WNV preparations were used as low and high internal controls. Next, rabbit anti-WNV polyclonal antibody (1:2000; Bioreliance) was added as detection antibody and after an incubation period of 1 h at RT, goat anti-rabbit IgG (H&L) alkaline phosphatase conjugate (1:2500; Calbiochem) was incubated at similar conditions. To visualize the ELISA signal, 100 µl pNPP substrate (Kem-en-Tech Diagnostics) per well was added for 1 h. The reaction was stopped with 1 M NaOH and the yellow colour was measured at OD₄₉₂ on a Bio-Tek microplate reader.

The inactivation/purification method disclosed above results in a yield that is about 10-12%, calculated from the starting material in the 20-liter wave bags, using the ELISA as a measuring tool for the concentration of particles.

### Example 4. Formulation of the purified West Nile Viruses

After determination of the concentration of inactivated West Nile Viruses from the previous example, the material is preferably further concentrated in a TFF setup with a hollow fiber filter unit (400 kDa, Spectrum) to reach a concentration of about 14,000 ELISA Units per ml (EU/ml), when the concentration is below that level. The same ELISA as described above is used for the determination of these units. If the determined concentration was above 14,000 EU/ml, the solution was preferably further diluted in formulation buffer without Tween-80 to reach the concentration of 14,000 EU/ml. Then, a 10x concentrated Tween-80 solution in 20 mM Tris, 110 mM NaCl, pH 7.4 was added to reach a final concentration of either 0.001% or 0.0005% Tween-80 in the resulting product.

To again reduce the bioburden level, the material was again filtered with a 0.22 µm stacked disc filter (Millipore) resulting in a WNV Drug Substance with about 14,000 EU/ml, which was stored at 4°C until further use.

Final handling was performed as follows. Whole-inactivated virus containing solution was further diluted in formulation buffer to different concentrations (1200 EU/ml, 4000 EU/ml and 12000 EU/ml) and subjected to a final sterilization filtration over a 0.22 µm stacked disc filter (Millipore). After this, the solution was adjuvated by the addition of an adjuvant.

Aluminum adjuvants are the most generally used adjuvants in both human and veterinary vaccines. These adjuvants have been used in practical vaccination for more than 60 years and are generally recognized as safe and as stimulators of Th2 immunity (Lindblad, 2004). These particular adjuvants, aluminum hydroxide (alum; Alhydrogel; Al(OH)₃) and aluminum phosphate (AlPO₄), are commonly used to increase the potency of vaccines. The major mechanisms responsible for adsorption of antigens to these adjuvants are ligand exchange and electrostatic attraction (Vogel and Hem, 2004). The stable adjuvated formulation of inactivated WNV is most preferably suitable for subcutaneous or intramuscular injection. Initial experiments demonstrated that the actual isoelectric point of WNV, important for electrostatic interaction of WNV to aluminum, is lower than 7.5. In addition, buffers with a pH of 5 and 6 had a profound effect on WNV, which was either visualised by an increase of OD₂₈₀ signal or by a decrease in E protein specific antibody binding (data not shown). Furthermore, it was found that purified whole-inactived WNV adsorbed to plastic and glass, thereby decreasing the WNV concentration in solution as measured with the WNV E protein specific ELISA. The addition of Bovine Serum Albumin (BSA) or Tween-80 to the formulation buffer prevented this adsorption of WNV. Since BSA would complicate the adjuvation process it was decided to proceed with Tween-80. Preferably, Tween-80 concentrations of ≥ 0.001% were used to prevent adsorption of WNV.

Based on these results, adjuvation of WNV to Al(OH)₃ was initially conducted with four different formulation buffers:
- 50 mM Tris, pH 7, 110 mM NaCl, 0.001% Tween-80
- 50 mM Tris, pH 7, 110 mM NaCl, 0.005% Tween-80
- 20 mM Tris, pH 8, 130 mM NaCl, 0.001% Tween-80
- 20 mM Tris, pH 8, 130 mM NaCl, 0.005% Tween-80

It should be noted that instead of NaCl, glycerine may also be used to obtain an isotonic formulation buffer. Adjuvation of WNV (ranging from 240 to 22,500 EU/ml) to alum (0.5 and 1 mg/ml) was successful in all four buffers to a similar extent; no unbound WNV was found in the supernatant of the adjuvated material (as determined using the E protein specific ELISA). Complete adjuvation was observed within 30 minutes. The successful binding of WNV to alum at pH 8 may be an indication that ligand exhange is more imporant for this adjuvation process than electrostatic interaction.

The effect of Tween-80 on the enveloped WNV is monitored in stability studies with drug substance (only WNV) and drug product (WNV adjuvated to alum).

Potency studies in mice demonstrate that Tween does not have an adverse effect on potency. In addition, initial in vitro development experiments using the ELISA for both drug substance and product measurements, indicated that particles released from WNV after treatment with detergent (mimicking the process of instability), do re-adjuvate to alum. Besides the increase in potency of an adjuvated product, this latter observation is another important argument for the use of alum as adjuvant for vaccine products, and preferably a WNV vaccine that is to be used in humans.

The addition of the aluminum hydroxide to the whole-inactivated and purified WNV batch resulted in a bulk WNV drug product, which was then filled and finished into a final drug product in transportable glass vials.

### REFERENCES

Arroyo J, Miller CA, Catalan J and Monath TP (2001) Yellow fever vector live-virus vaccines: West Nile Virus vaccine development. Trends Mol Med 7:350-354

Chang GJ, Davis BS, Hunt AR, Holmes DA and Kuno G (2001) Flavivirus DNA vaccines: current status and potential. Ann NY Acad Sci 951:272-285

Chang G-JJ, Kuno G, Purdy DE and Davis BS. (2004) Recent advancement in flavivirus vaccine development. Expert Rev Vaccines 3:199-220

Lindblad EB. (2004) Aluminium adjuvants - in retrospect and prospect. Vaccine 22:3658-3668

Malkinson M, Banet C, Khinich Y, Samina I, Pokamunski S and Weisman Y (2001) Use of live and inactivated vaccines in the control of West Nile fever in domestic geese. Ann N Y Acad Sci 951:255-261

Reyes-Del Valle J, Chavez-Salinas S, Medina F, and Del Angel RM (2005). Heat shock protein 90 and heat shock protein 70 are components of dengue virus receptor complex in human cells. J Virol 79:4557-4567

Vogel FR and Hem SL. (2004) "Immunologic adjuvants" in Vaccines; Ed. Plotkin SA, Orenstein WA and Offit PA. Elsevier Inc. p.69-79

Wang T, Anderson JF, Magnarelli LA, Wong SJ, Koski RA and Fikrig E (2001) Immunization of mice against West Nile Virus with recombinant envelope protein. J Immunol 167:5273-5277

## Claims

1. A method for the inactivation of a flavivirus, comprising subjecting said flavivirus to beta-propiolactone (BPL) for an appropriate period of time to yield a solution in which no live flavivirus can be detected.

2. A method according to claim 1, wherein said flavivirus is a West Nile Virus.

3. A method according to claim 1 or 2, wherein said flavivirus is grown on cultured cells in vitro.

4. A method according to claim 3, wherein the solution comprising the flaviviruses produced on said cells is treated with benzonase.

5. A method according to claim 4, wherein said benzonase treatment takes place before further processing of the solution.

6. A method according to any one of claims 1-5, wherein said method further comprises one or more of the steps of clarification and/or concentration/diafiltration.

7. A method according to any one of claims 1-6, wherein said BPL is present in a range of 0.01% to about 5%, preferably in a range of about 0.025% to about 1%, more preferably in a range of about 0.025% to about 0.5%, and most preferably in a concentration of about 0.1%.

8. A method according to any one of claims 1-7, wherein said inactivation by BPL takes place during a period of about 5-72 hr, preferably during a period of about 10-48 hr, more preferably during a period of about 18-44 hr, most preferably during a period of about 36-44 hr.

9. A method according to any one of claims 1-8, further comprising a step of binding said inactivated flavivirus to an affinity chromatography resin.

10. A method according to claim 9, wherein said affinity chromatography resin is Cellufine sulfate.

11. A method according to claim 9 or 10, further comprising the step of eluting the bound flavivirus by a high salt concentration in the elution buffer.

12. A method according to claim 11, wherein said high salt concentration is about 0.8 M to about 5 M NaCl, more preferably 1 M to about 2 M NaCl, even more preferably about 1.5 M NaCl.

13. A method according to any one of claims 1-12, further comprising a step of subjecting said inactivated flavivirus to a group separation chromatography step.

14. A method according to claim 13, wherein an affinity chromatography step preceeds said group separation chromatography step.

15. A method according to any one of claims 1-14, wherein said method further comprises one or more diafiltration steps.

16. A flavivirus obtained according to a method of any one of claims 1-15.

17. A West Nile Virus vaccine comprising a whole-inactivated West Nile Virus, wherein said West Nile Virus is formulated in a solution that is pH-controlled and further comprises a salt, a detergent and a pharmaceutically acceptable carrier.

18. A West Nile Virus vaccine according to claim 17, further comprising an adjuvant.

19. A West Nile Virus vaccine according to claim 18, wherein said adjuvant is an aluminum-based adjuvant.

20. A West Nile Virus vaccine according to claim 19, wherein said adjuvant is aluminum hydroxide.

21. A West Nile Virus vaccine according to any one of claims 17-20, wherein said salt is NaCl.

22. A West Nile Virus vaccine according to any one of claims 17-21, wherein said detergent is Tween-80.
